# EUROPEAN PATENT APPLICATION

(11) **EP 2 281 584 A1**
(43) Date of publication of application: **09.02.2011**
(21) Application number: 10181092.7
(22) Date of filing: 09.12.2003
(51) Int. Cl.: A61L 12/08, A45C 11/00, A45C 11/04, B65B 25/00

(54) **Contact lens packages containing additives**

(30) Priority: 23.12.2002 US 436109 P
(62) Divisional of application: 06076076.6
(71) Applicant: Johnson and Johnson Vision Care, Inc., Jacksonville, FL 32256 (US)
(72) Inventor: Peck, James, Maple Grove, MN 55369 (US); Dubey, Dharmesh, Jacksonville, FL 32256 (US); Tokarski, Michael, Ponte Vedra Beach, FL 32082 (US); Zhang, Qiang, Annandale, NJ 08801 (US); Li, Yufu, Bridgewater, NJ 08807 (US); Arnold, Steven, Sparta, NJ 07871 (US)
(74) Representative: Tunstall, Christopher Stephen

(57) **Abstract**

A package for storing medical devices in a solution comprising a molded base wherein the molded base comprises an additive, provided that the medical device is not a contact lens consisting of acqualfilcon A coated with polyHema, wherein the additive is PVP/maleic anhydride.

## Description

### RELATED APPLICATIONS

This application is a non-provisional filing of a provisional application, U.S. Pat. App. No.60/436,109, filed on December 23, 2002.

### FIELD OF THE INVENTION

This invention related to packages for storing contact lenses as well as methods of using and preparing these packages.

### BACKGROUND

Contact lenses have been used commercially to improve vision since the 1950s. At first contact lenses were made of hard materials, which were relatively easy to handle and package for use, but were uncomfortable for many patients. Later developments, gave rise to softer more comfortable lenses made of hydrophobic hydrogels, particularly silicone hydrogels. These lenses are very pliable, but due to this texture and their chemical composition, they present a number of problems with packaging.

Most contact lenses are packaged in individual blister packages having a bowl portion and a foil top, where the bowl portion is made from a hydrophobic material such as polypropylene. See U.S. Patent Nos. 4,691,820; 5,054,610; 5,337,888; 5,375,698; 5,409,104; 5,467,868; 5,515,964; 5,609,246; 5,695,049; 5,697,495; 5,704,468; 5,711,416; 5,722,536; 5,573,108; 5,823,327; 5,704,468; 5,983,608; 6,029,808; 6,044,966; and 6,401,915 for examples of such packaging, all of which are hereby incorporated by reference in their entirety. While polypropylene is resilient enough to withstand the sterilization steps of contact lens manufacture, this material has an affinity for contact lenses made of silicone hydrogels. When silicone hydrogels are packaged in polypropylene bowls, the lenses stick to the bowl and cannot be removed from the package without damaging the pliable lenses. Therefore is a need to prepare a contact lens package that has resilient properties, but does not stick to the final product. It is this need that is met by the following invention.

### BRIEF DESCRIPTION OF THE DRAWINGS

Figure 1 illustrates the data for Lens A in different packages
Figure 2 illustrates the data for Lens B in different packages
Figure 3 illustrates the data for Lens C in different packages

### DETAILED DESCRIPTION OF THE INVENTION

This invention includes a package for storing medical devices in a solution comprising, consisting essentially of, or consisting of, a molded base wherein the molded base comprises an additive, provided that the medical device is not a contact lens consisting of acqualfilcon A coated with polyHema.

As used herein a "medical device" is any device that is stored or packaged in a solution and is used to treat a human disease. Examples of medical devices include but are not limited to ophthalmic devices that reside in or on the eye. Ophthalmic devices includes but are not limited to soft contact lenses, intraocular lenses, overlay lenses, ocular inserts, and optical inserts. These devices can provide optical correction or may be cosmetic. The preferred medical devices of the invention are soft contact lenses made from silicone elastomers or hydrogels, which include but are not limited to silicone hydrogels, and fluorohydrogels. Soft contact lens formulations are disclosed in U.S. Pat. App. No. 60/318,536, entitled Biomedical Devices Containing Internal wetting Agents," filed on September 10, 2001 and its non-provisional counterpart of the same title, filed on September 6, 2002, US Patent No. 5,710,302, WO 9421698, EP 406161, JP 2000016905, U.S. Pat. No. 5,998,498, US Pat. App. No. 09/532,943, U.S. Patent No. 6,087,415, U.S. Pat. No. 5,760,100, U.S. Pat. No.5,776, 999, U.S. Pat. No. 5,789,461, U.S. Pat. No. 5,849,811, and U.S. Pat. No. 5,965,631. The foregoing references are hereby incorporated by reference in their entirety. The particularly preferred medical devices of the invention are soft contact lenses made from etafilcon A, genfilcon A, lenefilcon A, polymacon, balafilcon A, lotrafilcon A. and silicone hydrogels as prepared in U.S. Pat. No. 5,998,498, U.S. Pat. App. No. 09/532,943, a continuation-in-part of US Pat App. No. 09/532,943, filed on August 30, 2000, U.S. Patent No. 6,087,415, U.S. Pat. No. 5,760,100, U.S. Pat. No.5,776, 999, U.S. Pat. No. 5,789,461, U.S. Pat. No. 5,849,811, and U.S. Pat. No. 5,965,631. These patents as well as all other patent disclosed in this application are hereby incorporated by reference in their entirety. The more particularly preferred medical devices of the invention are soft contact lenses, balafilcon A, lotrafilcon A, galyfilcon A, senofilcon A, or those made as described in U.S. Pat. App. No. 60/318,536, entitled Biomedical Devices Containing Internal wetting Agents," filed on September 10, 2001 and its nonprovisional counterpart of the same title, filed on September 6, 2002. The most particularly preferred medical devices are soft contact lenses made from either galyfilcon A or senofilcon A.

The term "molded base" refers to any polymer, rubber, or plastic that can be formed into a receptacle for medical devices, where the size and shape of the base are determined by the device and other considerations known those who are skilled in the art of making or designing molded bases. For example molded bases may be individual blister packages, secondary packages, or hydrating trays. The molded base may be prepared from any number of materials provided that those materials are compatible with the chemical and physical properties of the device. Examples of suitable materials include but are not limited to polypropylene, polyethylene, nylons, olefin co-polymers, acrylics, rubbers, urethanes, polycarbonates, or fluorocarbons. The preferred materials are metallocenes polymers and co-polymers made of polypropylene, polyethylene, having a melt flow range of about 15 g/10 minutes to about 44 g/10 minutes as determined by ASTM D-1238. With respect to the shape of the molded base, examples of suitably shaped bases are disclosed in the following patents which are hereby incorporated by reference in their entirety, U.S. Patent Nos. D 458,023; 4,691,820; 5,054,610; 5,337,888; 5,375,698; 5,409,104; 5,467,868; 5,515,964; 5,609,246; 5,695,049; 5,697,495; 5,704,468; 5,711,416; 5,722,536; 5,573,108; 5,823,327; 5,704,468; 5,983,608; 6,029,808; 6,044,966; and 6,401,915. As in the cited references, the molded based is sealed about the cavity that encloses the contact lens. Flexible cover sheets can be made from can be an adhesive laminate of an aluminum foil and a polypropylene film or any other extruded or co-extruded film that can be sealed to the top surface of the flange in order to form a hermetic seal for the medical device and the solution. Further, the base can be formed by any of a number of known methods which include but are not limited to injection molding, transfer molding, skin packaging, blow molding, coinjection molding, film extrusion, or film coextrusion.

As used herein the term "additive" refers to a substance that is added to the polymer, rubber, or plastic prior to forming the molded base, where the material inhibits sticking, adherence, or adhesion of the medical device to the molded base. The additive is mixed with the remainder of the molded base material and amount of additive present by weight percentage based on the total weight of the molded base material is greater than about 0.25 to about 10 weight percent, preferably greater than about 0.25 to about 5 weight percent, most preferably about 0.25 to about 3 weight percent. The preferred additives are glycerol monostearate (2%), polyvinylpyrolidone (1% to 5%), polyvinylpyrolidone/maleic anhydride (1/1% to 5/5%), and succinic acid (5%). Polyvinylpyrolidinone has a variety of molecular weight ranges (as indicated by the KD#) and consistencies (flake, powdered/micronized). When PVP KD90 is used as an additive, it is preferred that it is powered/micronized.

The term "solution" refers to any liquid medium in which a medical device is stored. The preferred solutions are aqueous solutions contain physiological buffers. The particularly preferred solution is saline solution.

For example, if the medical device is a contact lens, it is preferred that the molded base is transparent to the degree necessary to permit visual inspection, UV sterilization or both. The preferred additives are glycerol monostearate present at about 2 weight percent, succininc acid present at about 5 weight percent, PVP KD90 present at about 1-5 weight percent, PVP/maleic anhydride present at about 1/1 to about 5/5 weight percent. If the inner surface of the medical device has a roughness of about 0.2 µm to about 4.5 µm, the preferred additives are maleic anhydride or PVP/maleic anhydride, most preferably maleic anhydride.

Further, the invention includes a method of reducing the adherence of a medical device to its packaging, comprising, consisting essentially of, or consisting of, storing said medical device in a solution in a package comprising, consisting essentially of, or consisting of, a molded base wherein said molded base comprises an additive, provided that the medical device is not a contact lens consisting of acqualfilcon A coated with polyhema. The terms molded base, medical device, solution and additive all have their aforementioned meanings and preferred ranges.

When soft contact lenses are prepared, the lenses cured to a hard disc and subsequently hydrated with water to give the non-steritized final product. During this hydration step, soft contact lenses often stick to the surface of the hydration chamber and it would useful to find a method of hydrating soft contact lenses which alleviates this problem.

To solve this problem, the invention includes a method of hydrating a contact lens comprising, consisting essentially of, or consisting of hydrating said lens in a molded base wherein said molded base comprises an additive. The terms molded base, medical device, solution and additive all have their aforementioned meanings. The preferred values for the medical device, the solution and the additive are as listed above. The preferred molded base is a square or a rectangle.

Other have tried to address the problem of a medical device adhering to its packaging. For example U.S. Pat App. No. 09/942,347, entitled "Textured Contact Lens Package," filed on August 29, 2001 and U.S. Pat. App. No. 10/183,133, entitled "Contact Lens Packages,"filed on June 26, 2002 disclose solutions to this problem. The disclosure of these applications are hereby incorporated by reference in their entirety. Even though those methods address this problem, it is contemplated by the inventors of this patent application that the additives of this invention may be incorporated into the packaging of each of the cited references.

In order to illustrate the invention the following examples are included. These examples do not limit the invention. They are meant only to suggest a method of practicing the invention. Those knowledgeable in contact lenses as well as other specialties may find other methods of practicing the invention. However, those methods are deemed to be within the scope of this invention.

### EXAMPLES

The following abbreviations are used below

| | |
|---|---|
| Ampacet 40604 | fatty acid amide |
| ATOFINA 3924CWZ | Finacene Nucleated polypropylene having a melt flow of 55g/10 minutes, ASTM D1238. This material contains an antistat and a lubricant |
| Atmer 163 | fatty alkyl diethanolamine Reg. No.107043-84-5 |
| Dow Siloxane MB50-321 | a silicone dispersion |
| Epolene E43-Wax, | maleic anhydride produced by Eastman Chemical |
| Erucamide | fatty acid amide Registry No. 112-84-5 |
| Exxon 1605 | Exxon Achieve, PP1605, a metallocene polypropylene having a melt flow of 32 g/10 minutes, ASTM D-1238 (L) |
| Exxon 1654 | Exxon Achieve, PP1654, a metallocene isotactic polypropylene having a melt flow of 16 g/10 minutes, ASTM D-1238 (L) |
| Fina EOD-001 | Finacene, a metallocene and isotactic polypropylene having a melt flow of 16g/90 minutes, ASTM D1238 |
| Flura | Registry No.7681-49-4 |
| Kemamide | fatty acid amide |
| Licowax | fatty acid amide |
| Mica | Registry No. 12001-26-2 |
| Nurcrel 535 & 932 | ethylene-methacrylic acid co-polymer resin Registry No. 25053-53-6 |
| Oleamide | fatty acid amide Registry No. 301-02-0 |
| polyHema | poly hydroxy ethylmethacylate having a molecular weight of greater than 1 MM Dalton |
| mPDMS | 800-1000 MW monomethacryloxypropyl terminated polydimethylsiloxane |
| Pluronic | polyoxypropylene-polyoxyethylene block co-polymer Registry No. 106392-12-5 |
| PVP | poly vinyl pyrrolidinone, wherein KD# refers to different known molecular weight distributions of poly vinyl pyrrolidinone |
| Simma 2 | 3-methacryloxy-2-hydroxypropyloxy)propylbis (trimethylsiloxy)methylsilane |
| Super-Floss anti block | slip/anti blocking agent, Registry No. 61790-53-2 |
| Tetronic | alkyoxylated amine 110617-70-4 |
| Zeospheres anti-block | slip/anti blocking agent |
| Lens Preparations | |
| Lens A | Acquafilcon A lenses coated with polyhema having a molecular weight of about 1,000,000. See U.S. Pat App. No. 09/957,299, entitled "Soft Contact Lenses," filed on September 20, 2001, Example 27. The coating method is disclosed in U.S. Pat. App. No. 09/921,192, entitled "Method for Correcting Articles by Mold Transfer," filed on August 2, 2001. |
| Lens B | Contact lenses prepared as described in U.S. Pat. App. No. 60/318,536, entitled Biomedical Devices Containing Internal wetting Agents," filed on September 10, 2001 and its non-provisional counterpart of the same title, filed on September 6, 2002, containing by weight percent 30% Simma 2, 19% mPDMS, 31% DMA, 6% PVP (MW 360,000), 0.8%EDGMA, 0.23% CGl81, 1.5% Norbloc, 11% PVP (MW 2,500), 0.02% Blue Hema, 0-2 ac PDMS, 29% t-amyl alcohol. |
| Lens C | Contact lenses prepared as described in U.S. Pat. App. No. 60/318,536, entitled Biomedical Devices Containing Internal wetting Agents," filed on September 10, 2001 and its non-provisional counterpart of the same title, filed on September 6, 2002, containing by weight percent 28% Simma 2, 31% mPDMS, 23.5% DMA, 7% PVP (MW 360,000), 1.5%TEDGMA, 0.98% CGl 1850,2.0% Norbloc, 6 HEMA, 0.02% Blue Hema. |

### Example 1

### Preparation of Packages with Different Additives

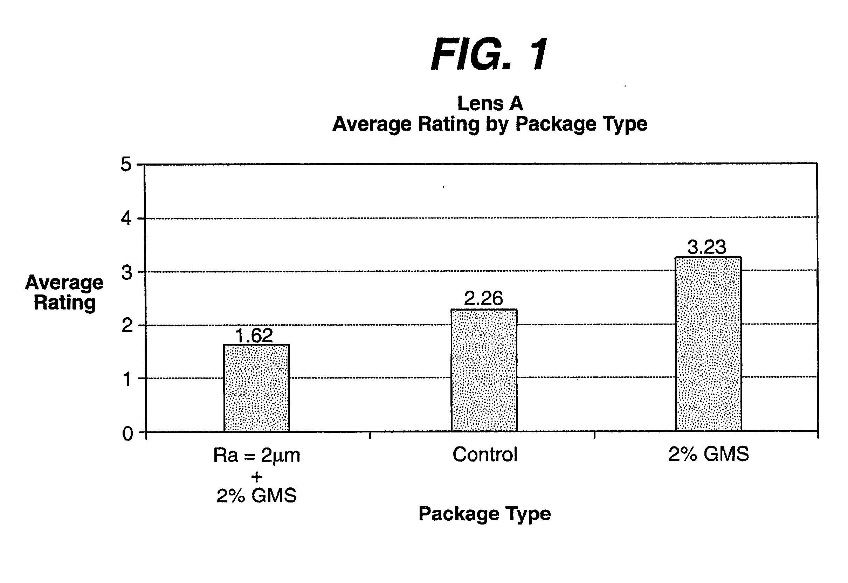

Additives (identity and amounts listed in Table 1) were mixed with polypropylene (listed below). The material was injection molded to form the base portion of a contact lens package. The configuration of the package is as illustrated in Figure1 of U.S. Pat No. 5,467,868 which is hereby incorporated by reference.

Contact lenses made from acquafilcon A coated with polyhema, a silicone hydrogel, were added to individual polypropylene blister packs having different additives containing 950µL of saline solution and then the blister pack was heat sealed with an flexible cover. Lenses were visually evaluated for adhesion to the package after sterilization. The flexible cover sheet was removed and the molded base is rotated or jiggled without spilling the saline solution while a contact lens is observed to determine if it is adhered to the inner surface of the molded base. Lenses that do not adhere are free floating and pass the test. If the lenses adhere to the molded base in any manner they fail the test. The addtitive, its weight percentage, the number of lenses that stuck to the package, and number of lenses that were free floating are displayed in Table 1. This example illustrates that glycerol monostearate is a superior additive.

**TABLE 1**

| Polypropylene | Additive | # tested | # stuck |
|---|---|---|---|
| Exxon 1605 | none | 12 | 12 |
| Exxon 1605 | calcium stearate | 36 | 36 |
| Exxon 1605 | 2% glycerol monostearate | 36 | 3 |
| Exxon 1654 | 2% glycerol monostearate | 84 | 2 |
| Exxon 1654 | none | 12 | 12 |
| Exxon Exxelor P1020 | none | 12 | 12 |
| Fina EOD-0011 | none | 12 | 12 |
| Fina EOD-0011 | 1% zinc stearate | 12 | 12 |
| Fina EOD-0011 | 3% zinc stearate | 12 | 12 |
| FINA 3924CW@ | antistat | 36 | 36 |

### Example 2

### Consumer Test

Packages containing 2% weight percent GMS and Exxon 1605 were prepared using the method of Example 1. Contact lenses of types A, B, and C were added to individual blister packages along with 950 µL of saline solution. The filed packages were heat sealed with flexible covers and sterilized. The packaged lenses were submitted to consumers. The consumers opened the packages and evaluated the lenses for ease of removal of the lens from the package using the following criteria and grading system
1-very easy removal-Lens comes out without any problems
2-easy removal-a couple of attempts to remove the lenses, but overall there were no real problems in removal
3-moderate removal- several tries before lens comes out, neither pleased or displeased
4-difficult removal-many tries to remove with finger or nail-removal is frustrating
5-very difficult removal-many tries to remove with a finger or nail, lens damage upon removal- very unacceptable

Figure 1 illustrates the testing results for a comparison of Lens A in a polypropylene package (control), Lens A in a package containing 2.0% GMS where the package has an average surface roughness (Ra) of about 2.0 µm, and Len A in a package containing 2.0% GMS. This figure shows that the roughened package containing GMS has the highest consumer rating.

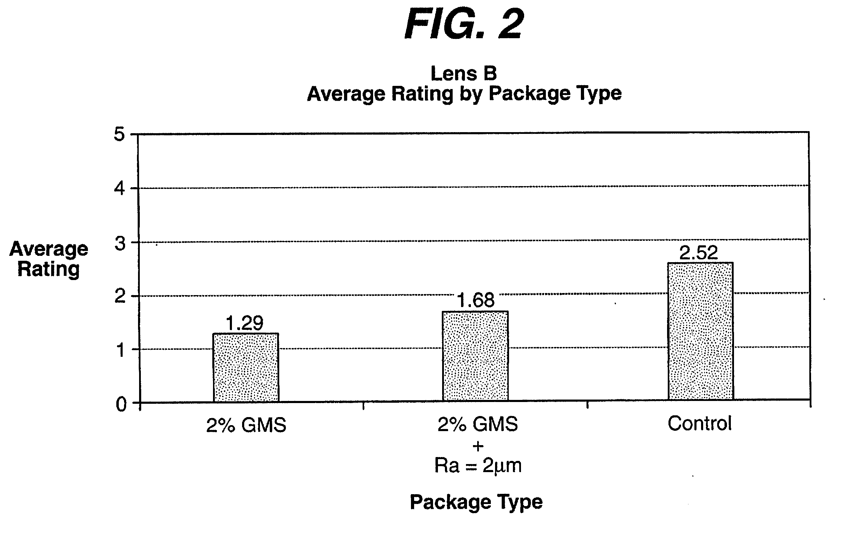

Figure 2 illustrates the testing results for a comparison of Lens B in a polypropylene package (control), Lens B in a package containing 2.0% GMS where the package has an average surface roughness (Ra) of about 2.0 µm, and Len B in a package containing 2.0% GMS. This figure shows that the package containing 2.0 %GMS has the highest consumer rating.

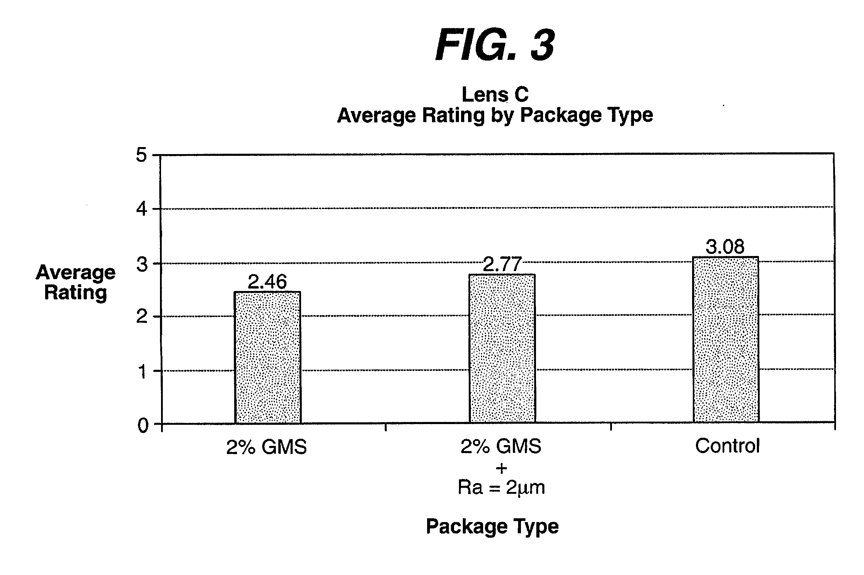

Figure 3 illustrates the testing results for a comparison of Lens C in a polypropylene package (control), Lens C in a package containing 2.0% GMS where the package has an average surface roughness (Ra) of about 2.0 µm, and Len C in a package containing 2.% GMS. This figure shows that the package containing 2.0 %GMS has the highest consumer rating.

### Example 3

### Preparation of Packages With Different Additives

The testing methods and preparations of Example 1 were repeated with different additives and lens types as per Table 2. If "(UP)" appears in an entry, that bowl of the blister is shaped as in U.S. Pat. No. D 458,023. When the term "Rough Bowl" appears, the inside surface of the bowl is roughened to an Ra of 0.5mm to 0.8mm.

**Table 2**

| Base Resin | Lens Type | Tested | Stuck | Additive |
|---|---|---|---|---|
| Exxon 1605 PP | Lens B | 15 | 13 | Calcium stearate (2%) |
| Exxon 1605 PP | Lens B | 120 | 0 | GMS (2%) |
| Exxon 1605 PP | Lens C | 30 | 0 | GMS (2%) |
| Exxon 1605 PP | Lens B | 15 | 12 | Dow Siloxane MB50-321 (10%) |
| Exxon 1605 PP | Lens B | 15 | 13 | Dow Siloxane MB50-321 (5%) |
| Exxon 1605 PP | Lens B | 57 | 50 | Ampacet 40604 99.6/.5 Erucamide |
| Ampacet 40604 PP | Lens B | 15 | 15 | Erucamide (5%) |
| Exxon 1605 PP | Lens B | 15 | 15 | Kemamide (Erucamide) (5%) |
| Exxon 1605 PP | Lens B | 15 | 12 | Superfloss anti-bock (2%) |
| Exxon 1605 PP | Lens B | 15 | 15 | Zeospheres anti-block (2%) |
| Exxon 1605 PP | Lens B | 15 | 14 | Superfloss anti-bock (2%) Oleamide (.2%) |
| Exxon 1605 PP | Lens B | 14 | 13 | Superfloss anti-bock (.2%) Oleamide (2%) |
| Exxon 1605 PP | Lens B | 15 | 15 | Talc (5%) |
| Exxon 1605 PP | Lens B | 15 | 13 | Calcium carbonate (5%) |
| Exxon 1605 PP | Lens B | 15 | 14 | Zinc stearate (5% hand blend) |
| Exxon 1605 PP | Lens B | 15 | 15 | Zinc stearate (5% machine blend) |
| Exxon 1605 PP | Lens B | 15 | 14 | ATP (Vitamin E) (5%) |
| Exxon 1605 PP | Lens B | 15 | 13 | Licowax (1%) |
| Exxon 1605 PP | Lens B | 15 5 | 14 | Polyethyleneglycol monolaurate (5%) |
| Exxon 1605 PP | Lens B | 15 | 15 | Mica (5%) |
| Exxon 1605 PP | Lens B | 175 | 8 | Succinic Acid (5%) |
| Exxon 1605 PP | Lens B | 15 | 13 | Succinic Anhydride (5%) |
| Exxon 1605 PP | Lens B | 118 | 22 | Epolene E-43 (20% machine blend) |
| Exxon 1605 PP | Lens B | 100 | 92 | Epolene E-43 (20% machine blend) |
| Exxon 1605 PP | Lens B | 127 | 52 | Epolene E-43 (10% hand blend) |
| Exxon 1605 PP | Lens B | 130 | 16 | Epolene E-43 (10% machine blend) |
| Exxon 1605 PP | Lens C | 15 | 6 | Epolene E-43 (10% machine blend) |
| Exxon 1605 PP | Lens B | 30 | 22 | Epolene E-43 (5% machine blend) |
| Exxon 1605 PP | Lens C | 15 | 3 | Epolene E-43 (5% machine blend) |
| Exxon 1605 PP | Lens B | 15 | 15 | Atmer 163 (1%) |
| Exxon 1605 PP | Lens B | 15 | 10 | MC (5%) |
| Exxon 1605 PP | Lens B | 30 | 2 | Boric Acid (5% hand blend) |
| Exxon 1605 PP | Lens B | 215 | 3 | Boric Acid (5% machine blend) |
| Exxon 1605 PP | Lens C | 15 | 0 | Boric Acid (5% machine blend) |
| Exxon 1605 PP | Lens B | 15 | 13 | Boric Acid (3% hand blend) |
| Exxon 1605 PP | Lens B | 15 | 15 | Boric Acid (2% hand blend) |
| Exxon 1605 PP | Lens B | 150 | 4 | Epolene E-43 (10% machine blend) |
| Exxon 1605 PP | Lens B | 50 | 9 | Epolene E-43 (10% machine blend) |
| Exxon 1605 PP | Lens B | 50 | 15 | Epolene E-43 (10% machine blend) |
| Exxon 1605 PP | Lens B | 50 | 35 | Epolene E-43 (10% machine blend) |
| Exxon 1605 PP | Lens B | 255 | 6 | PVP K90 (5.0%) |
| Exxon 1605 PP | Lens B | 98 | 31 | PVP K90 (2.5%) |
| Exxon 1605 PP | Lens B | 98 | 49 | PVP K90 (1.25%) |
| Exxon 1605 PP | Lens B | 20 | 6 | PVP K90 (1.0%) |
| Exxon 1605 PP | Lens B | 20 | 10 | PVP K90 (.75%) |
| Exxon 1605 PP | Lens B | 20 | 17 | PVP K90 (.5%) |
| Exxon 1605 PP | Lens C | 248 | 5 | PVP K90 (5.0%) |
| Exxon 1605 PP | Lens C | 39 | 0 | PVP K90 (10%) Blended down to 5% |
| Exxon 1605 PP | Lens C | 135 | 42 | PVP K90 (2.5%) |
| Exxon 1605 PP | Lens C | 135 | 54 | PVP K90 (1.25%) |
| Exxon 1605 PP | Lens C | 70 | 42 | PVP K90 (1.0%) |
| Exxon 1605 PP | Lens C | 70 | 50 | PVP K90 (.75%) |
| Exxon 1605 PP | Lens C | 70 | 60 | PVP K90 (.5%) |
| Exxon 1605 PP | Lens B | 15 | 14 | Nucrel 535 - 10.5% acid comonomer (2%) |
| Exxon 1605 PP | Lens B | 15 | 15 | Nucrel 925 -15% acid comonomer |
| (3%) | | | | |
| Exxon 1605 PP | Lens C | 15 | 14 | Nucrel 535 - 10.5% acid comonomer |
| (2%) | | | | |
| Exxon 1605 PP | Lens C | 15 | 14 | Nucrel 925 -15% acid comonomer |
| (3%) | | | | |
| Exxon 1605 PP | Lens B | 15 | 15 | 2% XNAP with Pluronic |
| Exxon 1605 PP | Lens C | 15 | 14 | 2% XNAP with Pluronic |
| Exxon 1605 PP | Lens B | 15 | 15 | Pluronic 1 % |
| Exxon 1605 PP | Lens C | 15 | 15 | Pluronic 1% |
| Exxon 1605 PP | Lens B | 15 | 11 | 1% Tetronic |
| Exxon 1605 PP | Lens C | 15 | 15 | 1% Tetronic |
| Exxon 1605 PP | Lens B | 15 | 15 | 1% Flura |
| Exxon 1605 PP | Lens C | 15 | 15 | 1% Flura |
| Exxon 1605 PP | Lens B | 30 | 23 | 2% Pluronic |
| Exxon 1605 PP | Lens C | 30 | 16 | 2% Pluronic |
| Exxon 1605 PP | Lens C | 77 | 0 | PVP K90 (5%) + Epolene E43 (5%) |
| Exxon 1605 PP | Lens B | 50 | 0 | PVP K90 (5%) + Epolene E43 (5%) |
| Exxon 1605 PP | Lens C | 62 | 0 | PVP K90 (5%) + Epolene E43 (1.5%) |
| Exxon 1605 PP | Lens B | 50 | 0 | PVP K90 (5%) + Epolene E43 (1.5%) |
| Exxon 1605 PP | Lens C | 65 | 0 | PVP K90 (2.5%) + Epolene E43 (1.25%) |
| Exxon 1605 PP | Lens B | 50 | 0 | PVP K90 (2.5%) + Epolene E43 (1.25%) |
| Exxon 1605 PP | Lens C | 115 | 10 | PVP K90 (1%) + Epolene E43 (1%) |
| Exxon 1605 PP | Lens B | 100 | 11 | PVP K90 (1%) + Epolene E43 (1%) |
| Exxon 1605 PP | Lens C | 30 | 0 | PVP K29/31 (5%) |
| Exxon 1605 PP | Lens C | 30 | 0 | PVP K60 (5%) |
| Exxon 1605 PP | Lens B | 50 | 0 | PVP K90 (1%) + Rough Bow1 (UP) |
| Exxon 1605 PP | Lens C | 50 | 0 | PVP K90 (1%) + Rough Bowl (UP) |
| Exxon 1605 PP | Lens B | 170 | 0 | Epolene E43 (1%) + Rough Bo-M |
| Exxon 1605 PP | Lens C | 200 | 0 | Epolene E43 (1%) + Rough Bowl |

The following is a list of embodiments which are or may be claimed:
Embodiment 1. A package for storing medical devices in a solution comprising a molded base wherein the molded base comprises an additive, provided that the medical device is not a contact lens consisting of acqualfilcon A coated with polyHema.
Embodiment 2. The package of embodiment 1 wherein the additive is selected from the group consisting of succinic acid, glycerol monostearate, PVP, and PVP/maleic anhydride.
Embodiment 3. The package of embodiment 1 wherein the additive is glycerol monostearate.
Embodiment 4. The package of embodiment 3 wherein glycerol monostearate is present at a concentration of greater than about 0.5 weight percent to about 5 weight percent.
Embodiment 5. The package of embodiment 3 wherein glycerol monostearate is present at a concentration of about 2 percent.
Embodiment 6. The package of embodiment 1 wherein the additive is PVP KD90.
Embodiment 7. The package of embodiment 6 wherein the PVP concentration is about 1% to about 5%.
Embodiment 8. The package of embodiment 6 wherein the PVP concentration is about 1.0%.
Embodiment 9. The package of embodiment 1 wherein the additive is PVP KD90/maleic anhydride.
Embodiment 10. The package of embodiment 9 wherein the PVP KD90/maleic anhydride concentration is about 1/1% to about 5/5%.
Embodiment 11. The package of embodiment 1 wherein the medical device is a contact lens which comprises balafilcon A, lotrafilcon A, galyfilcon, senofilcon, or lenses disclosed in U. S. Pat. App. No. 60/318,536, entitled "Biomedical Devices Containing Internal wetting Agents", filed on September 10, 2001 and its nonprovisional counterpart of the same title, filed on September 6, 2002.
Embodiment 12. The package of embodiment 11 wherein the contact lens comprises Simma 2 and mPDMS.
Embodiment 13. The package of embodiment 11 wherein the contact lens comprises Simma 2.
Embodiment 14. The package of embodiment 1 wherein the molded base comprises polypropylene.
Embodiment 15. The package of embodiment 1 further comprising a cavity formed in said molded base wherein said cavity comprises an inner surface, wherein said inner surface has an average roughness of about 0.5 µm to about 20 µm.
Embodiment 16. The package of embodiment 15 wherein the inner surface has an average roughness of about 1.8 µm to about 4.5 µm.
Embodiment 17. The package of embodiment 15 wherein the inner surface has an average roughness of about 1.9 µm to about 2.1 µm.
Embodiment 18. The package of embodiment 15 wherein the inner surface has an average roughness of about 0.5 µm to about 0.8 µm.
Embodiment 19. The package of embodiment 1 further comprising a cavity formed in said molded base wherein said cavity comprises an inner surface, wherein said inner surface has an average roughness of about 0.5 µm to about 20 µm and the additive is glycerol monostearate or PVP.
Embodiment 20. The package of embodiment 19 wherein the average roughness of the inner surface is about 0.5 µm to about 0.8 µn and the concentration of PVP is about 1%.
Embodiment 21. The package of embodiment 19 wherein the inner surface has an average roughness of about 1.9 µm to about 2.1 µm and the concentration of PVP is about 1%.
Embodiment 22. The package of embodiment 1 further comprising a cavity formed in said molded base wherein said cavity comprises an inner surface, wherein said inner surface has an average roughness of about 0.5 µm to about 20 µm and the additive is maleic anhydride or PVP/maleic anhydride.
Embodiment 23. The package of embodiment 22 wherein the average roughness of the inner surface is about 0.5 µm to about 0.8 µm and the concentration of PVP/maleic anhydride is about 1%.
Embodiment 24. The package of embodiment 22 wherein the inner surface has an average roughness of about 1.9 µm to about 2.1 µm and the concentration of PVP/maleic anhydride is about 1%.
Embodiment 25. The package of embodiment 22 wherein the average roughness of the inner surface is about 0.5 µm to about 0.8 µm and the concentration of maleic anhydride is about 1%.
Embodiment 26. The package of embodiment 22 wherein the inner surface has an average roughness of about 1.9 µm to about 2.1 µm and the concentration of maleic anhydride is about 1%.
Embodiment 27. A method of reducing the adherence of a medical device to its packaging, comprising storing said medical device in a solution in a package comprising a molded base wherein said molded base comprises an additive, provided that the medical device is not a contact lens consisting of acqualfilcon A coated with polyHema.
Embodiment 28. The method of embodiment 27 wherein additive is selected from the group consisting of succinic acid, glycerol monostearate, and PVP.
Embodiment 29. The method of embodiment 27 wherein the additive is glycerol monostearate.
Embodiment 30. The method of embodiment 27 wherein glycerol monostearate is present at a concentration of greater than about 0.25 weight percent to about 5 weight percent.
Embodiment 31. The method of embodiment 27 wherein glycerol monostearate is present at a concentration of about 2 percent.
Embodiment 32. The method of embodiment 27 wherein the additive is PVP KD90.
Embodiment 33. The method of embodiment 27 wherein the PVP is present at about 1% to about 5%.
Embodiment 34. The method of embodiment 27 wherein the contact lens comprises balafilcon A, lotrafilcon A, or lenses disclosed in U.S. Pat. App. No. 60/318,536, entitled "Biomedical Devices Containing Internal wetting Agents" filed on September 10, 2001 and its non-provisional counterpart of the same title, filed on September 6, 2002.
Embodiment 35. The method of embodiment 27 wherein the contact lens comprises Simma 2.
Embodiment 36. The method of embodiment 27 wherein the molded base comprises polypropylene.
Embodiment 37. The method of embodiment 27 further comprising a cavity formed in said molded base wherein said cavity comprises an inner surface, wherein said inner surface has an average roughness of about 0.5 µm to about 20 µm and the additive is glycerol monostearate or PVP.
Embodiment 38. The method of embodiment 37 wherein the average roughness of the inner surface is about 0.5 µm to about 0.8 µm and the concentration of PVP is about 1%.
Embodiment 39. The method of embodiment 37 wherein the inner surface has an average roughness of about 1.9 µm to about 2.1 µm and the concentration of PVP is about 1%.
Embodiment 40. The method of embodiment 27 further comprising a cavity formed in said molded base wherein said cavity comprises an inner surface, wherein said inner surface has an average roughness of about 0.5 µm to about 20 µm and the additive is maleic anhydride or PVP/maleic anhydride.
Embodiment 41. The method of embodiment 40 wherein the average roughness of the inner surface is about 0.5 µm to about 0.8 µm and the concentration of PVP/maleic anhydride is about 1 %.
Embodiment 42. The method of embodiment 40 wherein the inner surface has an average roughness of about 1.9 µm to about 2.1 µm and the concentration of PVP/maleic anhydride is about 1%.
Embodiment 43. The method of embodiment 40 wherein the average roughness of the inner surface is about 0.5 µm to about 0.8 µm and the concentration of maleic anhydride is about 1%.
Embodiment 44. The method of embodiment 40 wherein the inner surface has an average roughness of about 1.9 µm to about 2.1 µm and the concentration of maleic anhydride is about 1%.
Embodiment 45. A method of hydrating a contact lens comprising, consisting essentially of, or consisting of hydrating said lens in a molded base wherein said molded base comprises an additive.
Embodiment 46. The method of embodiment 45 wherein the additive is selected from the group consisting of succinic acid, glycerol monostearate, PVP, and PVP/maleic anhydride...
Embodiment 47. The method of embodiment 46 wherein the additives are present at a concentration of greater than about 0.25 weight percent to about 5 weight percent.
Embodiment 48. The method of embodiment 45 wherein the molded base further comprises a cavity formed in said molded base wherein said cavity comprises an inner surface, wherein said inner surface has an average roughness of about 0.5 µm to about 20 µm and the additive is maleic anhydride or PVP/maleic anhydride.

## Claims

1. A package for storing medical devices in a solution comprising a molded base wherein the molded base comprises an additive, provided that the medical device is not a contact lens consisting of acqualfilcon A coated with polyHema, wherein the additive is PVP/maleic anhydride.

2. A method of reducing the adherence of a medical device to its packaging, comprising storing said medical device in a solution in a package comprising a molded base wherein said molded base comprises an additive, provided that the medical device is not a contact lens consisting of acqualfilcon A coated with polyHema, wherein the additive is PVP/maleic anhydride.

3. The package of claim 1 wherein the additive is PVP KD90/maleic anhydride.

4. The package of claim 3 wherein the PVP KD90/maleic anhydride concentration is about 1/1% to about 5/5%.

5. The package of claim 1 or the method of claim 2 wherein the medical device is a contact lens which comprises balafilcon A, lotrafilcon A, galyfilcon, senofilcon, or lenses disclosed in U.S. Pat. App. No. 60/318,536, entitled "Biomedical Devices Containing Internal wetting Agents", filed on September 10, 2001 and its nonprovisional counterpart of the same title, filed on September 6, 2002.

6. The package of claim 5 wherein the contact lens comprises Simma 2 and mPDMS.

7. The package of claim 5 or the method of claim 2 wherein the contact lens comprises Simma 2.

8. The package of claim 1 or the method of claim 2 wherein the molded base comprises polypropylene.

9. The package of claim 1 or the method of claim 2 further comprising a cavity formed in said molded base wherein said cavity comprises an inner surface, wherein said inner surface has an average roughness of about 0.5 µm to about 20 µm.

10. The package of claim 9 wherein the inner surface has an average roughness of about 1.8 µm to about 4.5 µm.

11. The package of claim 9 wherein the inner surface has an average roughness of about 1.9 µm to about 2.1 µm

12. The package of claim 9 wherein the inner surface has an average roughness of about 0.5 µm to about 0.8 µm.

13. The package or method of claim 9 wherein the average roughness of the inner surface is about 0.5 µm to about 0.8 µm and the concentration of PVP/maleic anhydride is about 1%.

14. The package or method of claim 9 wherein the inner surface has an average roughness of about 1.9 µm to about 2.1 µm and the concentration of PVP/maleic anhydride is about 1%.
